# EUROPEAN PATENT SPECIFICATION

(11) **EP 2 049 157 B1**
(45) Date of publication and mention of the grant of the patent: **19.01.2011**
(21) Application number: 07785504.7
(22) Date of filing: 08.08.2007
(51) Int. Cl.: A61K 47/48

(54) **POLYMERIC CONJUGATES OF DOXORUBICIN WITH PH-REGULATED RELEASE OF THE DRUG AND A METHOD OF PREPARING**
POLYMERKONJUGATE VON DOXORUBICIN MIT PH-REGULIERTER FREISETZUNG DES ARZNEIMITTELS UND HERSTELLUNGSVERFAHREN
CONJUGUÉS POLYMÈRES DE LA DOXORUBICINE AVEC LIBÉRATION DU MÉDICAMENT CONTRÔLÉE PAR LE PH ET PROCÉDÉ POUR LES FABRIQUER

(30) Priority: 09.08.2006 CZ 20060505
(43) Date of publication of application: 22.04.2009
(73) Proprietor: Zentiva, k.s., Dolni Mecholupy 102 37 Praha 10 (CZ)
(72) Inventor: ETRYCH, Tomas, 152 00 Praha 5 (CZ); ULBRICH, Karel, 140 00 Praha 4 (CZ)
(74) Representative: Jirotkova, Ivana
(86) International application number: PCT/CZ2007/000077
(87) International publication number: WO 2008/017277

(56) References cited:
- WO-A-03/053473
- WO-A-2007/028347
- ETRYCH T ET AL: "New HPMA copolymers containing doxorubicin bound via pH-sensitive linkage: synthesis and preliminary in vitro and in vivo biological properties" JOURNAL OF CONTROLLED RELEASE, ELSEVIER, AMSTERDAM, NL, vol. 73, no. 1, 18 May 2001 (2001-05-18), pages 89-102, XP002248058 ISSN: 0168-3659 cited in the application
- GAO ET AL: "Colon-specific 9-aminocamptothecin-HPMA copolymer conjugates containing a 1,6-elimination spacer" JOURNAL OF CONTROLLED RELEASE, ELSEVIER, AMSTERDAM, NL, vol. 110, no. 2, 10 January 2006 (2006-01-10), pages 323-331, XP005222203 ISSN: 0168-3659
- DVORAK M ET AL: "High-molecular weight HPMA copolymer-adriamycin conjugates" JOURNAL OF CONTROLLED RELEASE, ELSEVIER, AMSTERDAM, NL, vol. 60, no. 2-3, 5 August 1999 (1999-08-05), pages 321-332, XP004362945 ISSN: 0168-3659
- TIJERINA M ET AL: "Correlation of subcellular compartmentalization of HPMA copolymer-Mce6 conjugates with chemotherapeutic activity in human ovarian carcinoma cells" PHARMACEUTICAL RESEARCH, NEW YORK, NY, US, vol. 20, no. 5, May 2003 (2003-05), pages 728-737, XP002453581 ISSN: 0724-8741
- KOPECEK J ET AL: "HPMA copolymer-anticancer drug conjugates: design, activity, and mechanism of action" EUROPEAN JOURNAL OF PHARMACEUTICS AND BIOPHARMACEUTICS, ELSEVIER SCIENCE PUBLISHERS B.V., AMSTERDAM, NL, vol. 50, no. 1, 3 July 2000 (2000-07-03), pages 61-81, XP004257180 ISSN: 0939-6411

## Description

### Technical Field

The invention concerns a new method of preparation of water-soluble polymeric cancerostatics that allow for targeted transport and regulated release of cytostatics in the organism, preferably in the tumor tissue and tumor cells. Polymeric cancerostatics are prepared directly via copolymerization with a monomer containing a cancerostatic in its structure. The use of polymeric conjugates focuses on targeted therapy of tumor diseases in humane medicine.

### Background Art

The development of new pharmacologically-potent substances, including cancerostatics, has been more and more focusing on new forms that allow for specific action of the active drug only in a certain tissue or even in a certain cell type. Targeted drugs find their use especially in the fields where side effects of the active ingredient can result in damage of healthy parts of the organism. Preparation of targeted drugs involves more and more the use of macromolecules - polymers, both natural and synthetic. In the past, a large number of polymeric conjugates of cancerostatics was prepared and studied and it was demonstrated that in most cases, it is necessary to ensure release of the original low-molecular cytotoxic substance from its polymeric form for the polymeric drug form to be pharmacologically effective. Of course, connecting a cytostatic to a water-soluble polymer via a chemical bond also allows for a radical increase of solubility of insoluble or low-soluble drugs and significantly decreases their toxicity. Last but not least, the higher molecular weight of the polymers prevents fast release of the drug from the organism via glomerular filtration and, thus, ensures prolonged time of its circulation in blood and retention in the organism, and, hence, higher bioavailability of the drug. It is advantageous to ensure the release of the cytostatic from the polymeric carrier via a biodegradable spacer, used to connect the drug to the polymer, the degradation of which in the target tissue leads to targeted and regulated release of the drug in the tissue. An important group of the above described drugs is polymeric drugs prepared on the basis of copolymers of *N*-(2-hydroxypropyl)methacrylamide (HPMA). A review of the hitherto obtained results in this field is very well elaborated in G.S. Kwon and in J. Kopecek et al. [Kopecek et al. 2000, Kwon 2005]. Recently, studies were published on preparation and working of polymeric drugs in which cancerostatic doxorubicin is bound to a polymeric carrier based on HPMA copolymers via a hydrolytically instable hydrazone bond [Etrych et al. 2001 and 2002, Rihova et al. 2001, Ulbrich et al. 2003, 2004a, 2004b] and the substances were patented [Ulbrich et al.]. The described drugs showed a significant decrease of side effects on the organism, especially the toxic ones, and simultaneously a significant increase of antitumor effectiveness compared to the commonly used low-molecular cytostastics [Rihova et al. 2001, Kovar et al. 2004, Hovorka et al. 2002].

Synthesis of such conjugates was performed first by a polymer-analogous reaction of polymeric 4-nitrophenyl esters (ONp) with hydrazine and later by copolymerization of HPMA with mathacryloylated hydrazides protected with *N*-Boc (*terc*-butyloxycarbonyl) group. Later, a new method of preparation of polymeric conjugates was developed which included preparation of the 6-(methacryloylamino)hexanoylhydrazine monomer and its copolymerization with HPMA. The method represented a significant progress in synthesis and allowed for precise regulation of the molecular weight of the polymeric precursors and the final product [Etrych patent]. In all the mentioned methods, the drug was bound to the polymeric precursor containing free hydrazide groups via polymer-analogous reaction.

Conjugates of a drug and HPMA containing a 1,6-elimination spacer were syntesited by radical copolymerization of HPMA with a 9-aminocamptothcein containing monomer [Gao et al. 2006]. The object of this invention is a new method of preparation of polymeric cytostatics based on HPMA copolymers with doxorubicin bound via a pH-labile hydrazone bond to a polymeric carrier. The method that we propose allows incorporating the drug in the structure of polymeric conjugate directly via copolymerization of HPMA with a monomer containing doxorubicin, connected to the polymerizable group with a hydrazone bond through a spacer, i.e. without the need for subsequent reaction of binding the drug spacer to the polymeric precursor. The use of the mentioned monomer in the synthesis allows to prepare polymeric conjugates with precisely defined structure of the polymeric chain that contains only monomeric units of HPMA and monomeric units carrying the hydrazone-bound drug.

### Disclosure of Invention

The invention consists essentially in a method of preparation of a polymeric conjugate of a HPMA copolymer with doxorubicin bound to the polymer via various spacers containing hydrolytically cleavable hydrazone bonds. The method consists of two-step synthesis involving synthesis of monomers, namely HPMA and methacryloylated derivatives of amino acids and oligopeptides, terminated with doxorubicin connected via the hydrazone bond, and direct synthesis of polymeric conjugates via copolymerization with the mentioned monomer containing the cancerostatic doxorubicin bound by a covalent hydrazone bond.

The polymeric drug prepared according to the invention is characterized by the fact that its structure is constituted by a hydrophilic water-soluble copolymer containing units of HPMA and units of a methacryloylated derivative of amino acids or oligopeptides, terminated with doxorubicin connected to the amino-acid or oligopeptide residues (spacers) via a pH-sensitive hydrolytically-cleavable hydrazone bond. The spacers can be constituted by individual amino acids, oligopeptides or other structures, allowing their termination with the hydrazide group and connection of doxorubicin to the same by hydrazone bond. The content of comonomeric (Dox containing) units in the copolymer can be from 0.5 to 10 mol. %. The copolymer does not contain any other poorly defined units, e.g. methacryloylated hydrazides.

The polymer with the chemically-bound cytostatic is stable during circulation in the bloodstream, the hydrazone bond between doxorubicin and the polymer is relatively stable under physiological conditions of the bloodstream (pH 7.4). After extravasation and entrapment in tumors, the molecularly-dissolved conjugate penetrates into individual tumor cells via pinocytosis and, due to the decrease of pH from the extracellular pH 7.4 to intracellular pH 5 to 6, hydrolysis of the hydrazone bond, release of cytostatic in the target cell and hence activation of its cytotoxic effect should take place. The feasibility of the above proposed mechanism of action of the polymeric drugs according to the invention is demonstrated by experiments of modeled release of doxorubicin from the polymeric carrier. The results of siad tests are presented in the experimental part of the application.

***Synthesis of monomers*** starts from synthesis of the HPMA monomer via the method described earlier [Ulbrich 2000]. Synthesis of methacryloyl(aminoacyl)hydrazides differing in the structure of the acyl component is very similar in all the prepared monomers and is performed using the procedure described earlier [Ulbrich patents, Etrych patent]. This synthesis starts from methacryloylation of the methyl ester of the hydrochloride of the respective amino acid or oligopeptide with methacryloylchloride, performed in dichloromethane in the presence of anhydrous sodium carbonate. The resulting product is converted to a methacryloylated aminoacylhydrazine by hydrazinolysis of the respective methyl ester with hydrazine hydrate, performed in a solution in methanol or 2-propanol. Preferably, the aminoacyl in the methacryloyl(aminoacyl)hydrazines can be glycyl, glycylglycyl, β-alanyl, 6-aminohexanoyl, 4-aminobenzoyl or a complex acyl derived from the oligopeptides GlyPheGly, GlyLeuGly or GlyPheLeuGly. As an example of synthesis of a methacryloyl(aminoacyl)hydrazine, synthesis of 6-methacroyl(aminohexanoyl)hydrazide is presented in Example 1.

Preparation of (methacryloylamino)acylhydrazide-doxorubicins starts from the binding reaction of doxorubicin hydrochloride to methacryloyl(aminoacyl)hydrazines producing the hydrazone bond. The reaction is preferably performed in methanol under catalysis with a defined amount of acetic acid with addition of an inhibitor. The reaction can be performed also in dimethylsulfoxide, dimethylformamide, dried ethanol or dimethylacetamide. If solvents other than methanol are used, the reaction proceeds well but the yields are lower. The influence of the spacer structure on the course of the binding reaction is minimal. For achieving the optimal yield of the bond and minimal content of unbound doxorubicin, it is important in any case to adhere to the concentrations of the reactants and acetic acid in the reaction mixture: 19 mg/ml concentration of doxorubicin (DOX), 51 mg/ml concentration of acetic acid. The optimal reaction time is 24 h at 25 °C. The above specified conditions are optimal, resulting in maximal yields. The reaction can be performed also under slightly modified reaction conditions, adjusted to the type of the solvent used as well as that of (methacryloylamino)acylhydrazide. If a lower DOX concentration is used (10 mg/ml), it is necessary to work at higher temperature (up to 35 °C), the concentration of acetic acid can be decreased down to 35 mg/ml, or alternatively the reaction time can be extended (up to 28 hours). At a higher DOX concentration (30 mg/ml) it is advantageous to increase the concentration of acetic acid up to max. 60 mg/ml and to shorten the reaction time to 20 hours, or alternatively to decrease temperature to 20 °C. For removing the free drug from the product a small addition of the poly(HPMA-co-MA-AH-NHNH₂) polymer can be preferably used, the hydrazide groups of which will bind the unreacted doxorubicin (DOX). The reaction mixture is subsequently purified by gel filtration, preferably in a LH-20 column in methanol. After concentrating, the monomeric DOX derivative fraction is isolated by precipitation in diethyl ether. Preparations of 6-(methacryloylamino)hexanoylhydrazide-doxorubicin and of methacroylglycylphenylalanylleucylglycylhydrazide-doxorubicin are presented as examples of synthesis of (methacryloylamino)acylhydrazide-doxorubicins in the experimental part.

***Synthesis of polymeric conjugates of doxorubicin** -* HPMA copolymers with methacryloylated derivatives of amino acids and oligopeptides, terminated with doxorubicin connected via the hydrazone bond, is based on direct radical copolymerization of HPMA with corresponding methacryloylated DOX derivatives of formula II

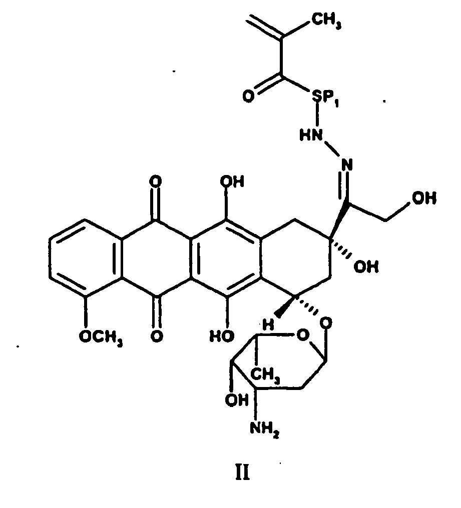

The polymerization is performed in solution using methanol, ethanol, dimethylsulfoxide or dimethylformamide as the polymerization medium. The polymerization is initiated with heat-degradable radical polymerization initiators based on azo or peroxy initiators. Azobis(isobutyronitrile) (AIBN), azobis(isocyanovaleric acid) (ABIC) or diisopropylpercarbonate (DIP) can be preferably used. The polymerization temperature depends on the respective initiator and the solvent used (50 to 60 °C for AIBN, ABIC in methanol, ethanol, DMF and DMSO, 40 to 50 °C for DIP). A usual time of polymerization is 15 to 24 hours. Preparation of all polymeric conjugates via radical polymerization is analogous; for example, copolymerization of HPMA with a methacryloylated DOX derivative is presented in Example 3. Compared to the earlier used polymer-analogous binding reaction of DOX to the polymeric precursor poly(HPMA-co-MA-AH-NHNH₂), the direct copolymerization of HPMA with a methacryloylated DOX derivative results in exactly defined polymeric conjugates.

***Polymeric conjugate*** is a copolymer of HPMA with methacryloylated derivatives of amino acids and oligopeptides, terminated with doxorubicin, connected with the hydrazone bond, of formula I 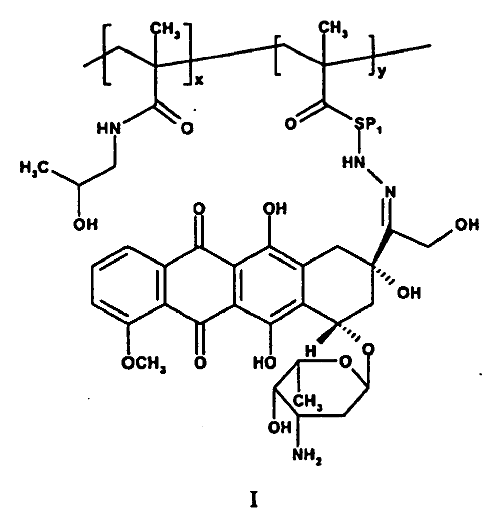 being **characterized in that** it contains 90 to 99.5 mol. % of HPMA and 10 to 0.5 mol. % of (methacryloylamino)acylhydrazide-doxorubicin units.

There has been introduced an abbreviation for the spacers in the side chains of the conjugates and monomers in the structural schemes herein - SP₁ is the aminoacyl in methacryloylacylhydrazide-doxorubicins, e.g. glycyl, glycylglycyl, β-alanyl, 6-aminohexanoyl (AH), 4-aminobenzoyl or a complex acyl derived from the oligopeptides GlyPheGly, GlyLeuGly, GlyLeuPheGly and GlyPheLeuGly.

### Brief Description of Drawings

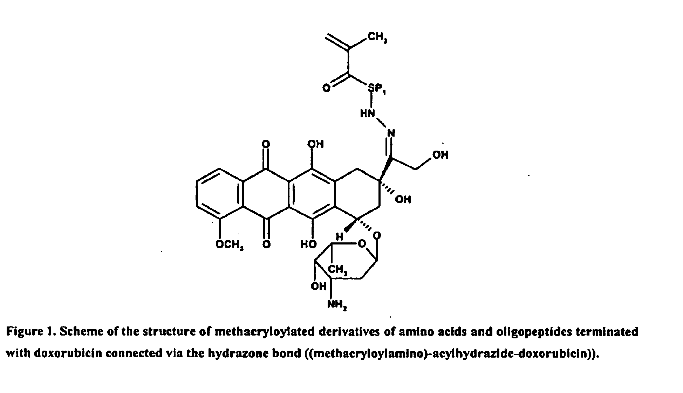

Figure 1 represents a scheme of structure of the methacryloylated derivatives of amino acids and oligopeptides, terminated with doxorubicin connected via the hydrazone bond ((methacryloylamino)-acylhydrazide-doxorubicin)).

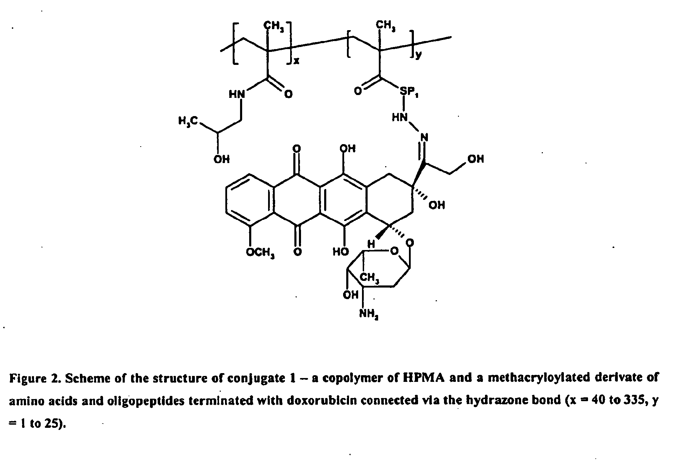

Figure 2 represents a scheme of structure of conjugate 1 - copolymer of HPMA and a methacryloylated derivative of amino acids and oligopeptides, terminated with doxorubicin connected via the hydrazone bond (x = 40 to 335, y = 1 to 25).
Figure 3 represents a graph of release rate of DOX from polymeric conjugate 1 and from the polymeric conjugate prepared via polymer-analogous reaction (patent Etrych) in a buffer with pH 5 (model of intracellular environment).
Figure 4 represents a graph of release rate of DOX from polymeric conjugate 1 and from the polymeric conjugate prepared via polymer-analogous reaction (patent Etrych) in a buffer with pH 7.4 (model of bloodstream).

### Examples

### Example 1: Synthesis of monomers

HPMA was prepared according to the procedure that was described earlier [Ulbrich et al. 2000]. Elementary analysis: calculated 58.8 % C, 9.16 % H, 9.79 % N; found 58.98 % C, 9.18 % H, 9.82 % N. The product was chromatographically pure.

6-(methacryloylamino)hexanoylhydrazide (*N*¹-(6-hydrazino-6-oxohexyl)-2-methylacrylamide) (MA-AH-NHNH₂) was prepared according to the procedure that was described earlier [Ulbrich patents, Etrych patent].

Methacroylglycylphenylalanylleucylglycylhydrazide (MA-Gly-D,L-PheLeuGly-NHNH₂) was prepared according to the procedure that was described earlier [Etrych patent].

### 6-(Methacryloylamino)hexanoylhydrazide-doxorubicin (MA-AH-NHN=DOX)

6-(Methacryloylamino)hexanoylhydrazide (40 mg, 0.188 mmol) was dissolved in 6 ml of methanol at room temperature. The solution was poured into a reaction vessel in which doxorubicin.HCl (115 mg, 0.198 mmol) was placed and the suspension was stirred vigorously. 310 µl of acetic acid was added to the suspension and reaction mixture was stirred at 25 °C for 24 hours. The reaction process was monitored by TLC - Silicagel 60 F₂₅₄ plates (methanol:chloroform:acetic acid 2:8:1, Rf(DOX) = 0.75, Rf(MA-AH-NHN=DOX)= 0.9). During the course of the reaction, the suspension was gradually dissolved and the solution was homogenous after 20 hours of reaction. After 24 hours, 100 mg of the poly(HPMA-co-MA-AH-NHNH₂) copolymer was added to the homogenous mixture (to bind the residual free DOX) and the reaction mixture was stirred at room temperature for another 4 hours. The product was purified from polymeric and low-molecular impurities by means of gel chromatography in a column (30 cm x 30 cm) filled with Sephadex LH-20 in methanol. The low-molecular fraction was concentrated to 2 ml and the product was precipitated into 30 ml of diethyl ether. The product was sucked off, washed with a small amount of diethyl ether, and dried in vacuo until constant weight. The yield was 110 mg of the product (79 %) with the melting point 172 to 175 °C. TLC (methanol:chloroform:acetic acid 2:8:1): one stain at Rf= 0.9. MALDI-TOF MS: 762 (M+Na).

Methacroylglycylphenylalanylleucylglycylhydrazide-doxorubicin (MA-GFLG-NHN=DOX) Preparation of MA- GFLG-NHN=DOX was performed under similar conditions as in the case of MA-AH-NHN=DOX. Methacroylglycylphenylalanylleucylglycylhydrazide (122 mg, 0.258 mmol) was dissolved in 8.2 ml of methanol at room temperature. The solution was poured into a reaction vessel in which doxorubicin.HCl (157 mg, 0.271 mmol) was placed and the suspension was stirred vigorously. 420 µl of acetic acid was added to the suspension and reaction mixture was stirred at 25 °C for 24 hours. The course of the reaction was monitored by TLC - Silicagel 60 F₂₅₄ plates (methanol:chloroform:acetic acid 2:8:1, Rf(DOX)= 0.75, Rf(MA-GFLG-NHN=DOX)= 0.95). During the course of the reaction, the suspension was gradually dissolved and the solution was homogenous after 19 hours of reaction. After 24 hours, 130 mg of the poly(HPMA-co-MA-AH-NHNH₂) copolymer was added to the homogenous mixture (to bind residual free DOX) and the reaction mixture was stirred at room temperature for another 4 hours. The product was purified from polymeric and low-molecular impurities by means of gel chromatography in a column (30 cm x 30 cm) filled with Sephadex LH-20 in methanol. The low-molecular fraction was concentrated to 2.5 ml and the product was precipitated into 40 ml of diethyl ether. The product was sucked off, washed with a small amount of diethyl ether, and dried in vacuo until constant weight. The yield was 210 mg of the product (78 %) with the melting point 179 to 182 °C. TLC (methanol:chloroform:acetic acid 2:8:1): one stain at Rf= 0.95. MALDI-TOF MS: 1023 (M+Na).

### Example 2: Synthesis of a polymeric conjugate - Conjugate 1 - a copolymer of HPMA with MA-AH-NHN=DOX

The poly(HPMA-co-MA-AH-NHN=DOX)] copolymer was prepared via solution radical copolymerization of HPMA and MA-AH-NHN=DOX in methanol at 60 °C.

840 mg of HPMA and 165 mg of MA-AH-NHN=DOX (18 w. % of the monomers) was dissolved in 5.7 ml of methanol and 67 mg of ABIN (1.2 w. %) was added to the solution. After filtration, the polymerization mixture was charged, in an argon atmosphere, into a polymerization reactor (20 ml volume) situated in a thermostat. Nitrogen was introduced above the surface for several additional minutes. The temperature of the polymerization mixture was set at 60 °C and the polymerization proceeded under stirring (50 rpm) in the nitrogen atmosphere. The polymerization mixture was taken out of the thermostat after 22 hours, cooled to room temperature in a bath, and the polymer was isolated by precipitation with ethyl acetate (100 ml in total). The precipitated polymer was isolated by filtration through S4 fritted glass. The precipitate was washed with ethyl acetate and dried at room temperature in vacuo in a membrane pump for about 1 hour. The polymeric product was purified from low-molecular impurities and unbound drug using gel chromatography in a column filled with Sephadex LH-20 in methanol. The polymeric fraction was entrapped, concentrated in a vacuum rotatory evaporator until the volume of 5 ml, and the copolymer was isolated by precipitation with 50 ml of ethyl acetate. The product was dried until constant weight.

The total DOX content was determined spectrally. *M̅_{w}* and *M̅ₙ* were determined by liquid chromatography (LC AKTA) with detection based on light dispersion (DAWN DSP Multiangel detector, Wyatt).

Characterization of the polymeric drug: the yield of polymerization reaction: 750 mg (75 %), total DOX content 10.8 weight %, free DOX content 0.35 % of the total DOX content, molecular weight *M̅_{w}* = 34000, polydispersity index *M̅_{w}*/*M̅ₙ =* 1.72.

### Example 3: Synthesis of a polymeric conjugate - Conjugate 2 - a copolymer of HPMA with MA-AH-NHN=DOX

The poly(HPMA-co-MA-AH-NHN=DOX)] copolymer was prepared via solution radical copolymerization of HPMA and MA-AH-NHN=DOX in methanol at 60 °C by the same method as in Example 2, with the difference that the composition of polymerization mixture was as follows: 770 mg of HPMA, 235 mg of MA-AH-NHN=DOX, 5.7 ml of methanol, 67 mg of ABIN (1.2 weight %). Isolation and purification of the product was performed via the same method as in Example 2. Characterization of the polymeric drug: the yield of polymerization reaction: 740 mg (74 total DOX content 16.5 weight %, free DOX content 0.45 % of the total DOX content, molecular weight *M̅_{w}* = 32800, polydispersity index *M̅_{w} l M̅ₙ =* 1,78.

### Example 4: Synthesis of a polymeric conjugate - Conjugate 3 - a copolymer of HPMA with MA-GFLG-NHN=DOX

The poly(HPMA-co-MA-GFLG-NHN=DOX) copolymer was prepared via solution radical copolymerization of HPMA and MA-GFLG-NHN=DOX in methanol at 60 °C by the same method as in Example 2, with the difference that the composition of polymerization mixture was as follows: 700 mg of HPMA, 183 mg of MA-GFLG-NHN=DOX, 5 ml of methanol, 64 mg of ABIN (1.3 weight %). Isolation and purification of the product was performed via the same method as in Example 2. Characterization of the polymeric drug: the yield of polymerization reaction: 670 mg (76 %), total DOX content 10.5 weight %, free DOX content 0.32 % of the total DOX content, molecular weight *M̅_{w}* = 34800, polydispersity index *M̅_{w}* / *M̅ₙ =* 1,82.

### Example 5: Release of doxorubicin from grafted polymeric conjugates

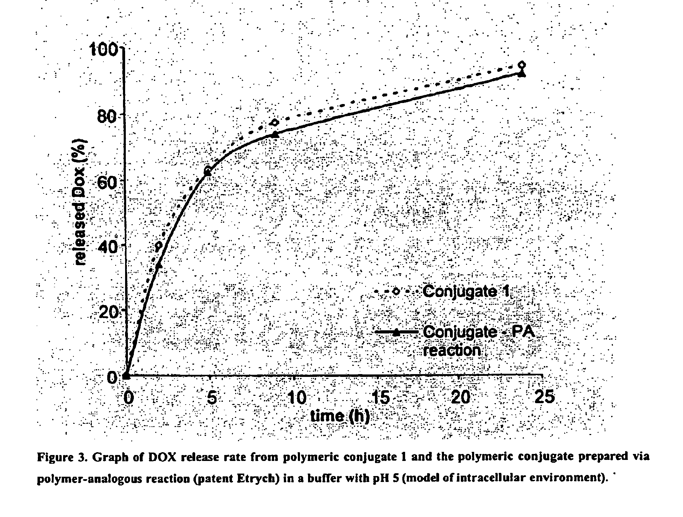

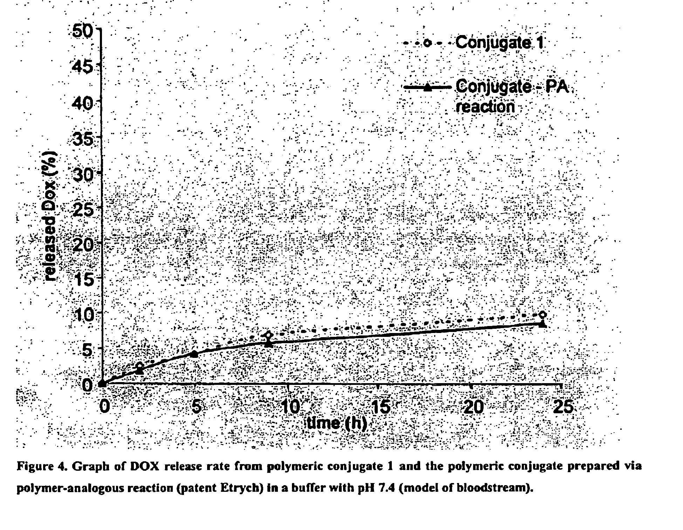

The amounts of doxorubicin released from polymeric conjugates after their incubation in a phosphate buffer with pH 5.0 (0.1 M phosphate buffer containing 0.05 M NaCl), modeling the intracellular environment, and in a phosphate buffer with pH 7.4, modeling the bloodstream environment, were measured. The amount of released DOX in the incubation solution was determined bymeans of HPLC (Shimadzu). In predetermined intervals, 50 µl of the incubation solution were sampled and analyzed in a TSKGel G 3000xl column, isocratic flow 0.5 ml/min of the mobile phase composed of the methanol:acetate buffer pH 6.5 mixture (80 : 20 vol. %). The amount of DOX was calculated from the areas of peaks of free and bound DOX (UV-VIS detection at 488 nm). After incubation of the conjugates (5 mg/ml concentration) in the physiologic environment at 36 °C (phosphate buffer, pH 7.4), only a small amount of the drug is released (up to 10 %/24 hours) (Figure 4); on the contrary, the release rate of DOX from the grafted polymeric conjugates, and hence the activation rate of the cytotoxic drug, is high in the slightly acidic environment at pH 5.0 (Figure 3). The drug release rates of drug release at pH 7.4 and pH 5 from the polymeric conjugates prepared via direct copolymerization using 6-(methacryloylamino)acylhydrazide-doxorubicines are fully comparable with those detected for the hydrazone conjugates prepared via polymer-analogous reaction (PA reaction) [Etrych patent]. The results of measurements of *in vitro* release rate of the active drug from the polymeric carrier in the environment modeling the blood medium (in transport of the drug through the organism) and in intracellular environment of the target cell confirm suitability of using the proposed polymeric cytostatic for tumor-specific therapies.

### Literature:

Etrych, T., Jelinkova, M., Rihova, B. and Ulbrich K., New HPMA copolymers containing doxorubicin bound via pH sensitive linkage. Synthesis, in vitro and in vivo biological properties. J. Controlled Release 73, 89-102 (2001*).*
T. Etrych, P. Chytil, M. Jelinkova, B. Rihova, K. Ulbrich, Synthesis of HPMA Copolymers Containing Doxorubicin Bound via a Hydrazone Linkage. Effect of Spacer on Drug Release and in vitro Cytotoxicity. Macromolecular Biosci. 2, 43-52 (2002*)*
Etrych T., Chytil P., Pechar M., Studenovsky M., Rihova B., Ulbrich K.: A method of preparing of polymeric conjugates of doxorubicin with pH-controlled release of the drug, CZ PV 2005-558
Gao, S., Lu, Z., Petri, B., Kopeckova, P., Kopecek, J.: Colon-specific 9-aminocamptothecin-HPMA copolymer conjugates containing a 1,6-elimination spacer. J. Controlled Release 110, 323-331 (2006).
O. Hovorka, T. Etrych, M. Subr, J. Strohalm, K. Ulbrich, B. Rihova, Differences in the Intracellular Fate of Free and Polymer-Bound Doxorubicin. J. Controlled Release 80, 101-117 (2002)
J. Kopecek, P. Kopeckova, T. Minko, Z. Lu, HPMA Copolymer-Anticancer Drug Conjugates: Design, Activity, and Mechanism of Action. Europ. J. Pharm. Biopharm. 50, 61 - 81 (2000)
M. Kovar, L.Kovar, V. Subr, T. Etrych, K. Ulbrich, T. Mrkvan, J. Loucka and B. Rihova, HPMA Copolymers Containing Doxorubicin Bound by Proteolytically or Hydrolytically Cleavable Bond: Comparison of Biological Properties In Vitro. J. Controlled Release 99, 301-314 (2004)
G.S. Kwon, Polymeric Drug Delivery Systems, Series: Drugs and the Pharmaceutical Sciences, Vol. 148, Dekker, Marcel Incorporated, 2005
B. Rihova, T. Etrych, M. Pechar, M. Jelinkova, M. Stastny, O. Hovorka, M. Kovar, K. Ulbrich, Doxorubicin Bound to a HPMA Copolymer Carrier Through Hydrazone Bond is Effective also in a Cancer Cell Line with a Limited Content of Lysosomes. J. Controlled Release 74, 225-232 (2001)
K. Ulbrich, V. Subr, J. Strohalm, D. Plocova, M. Jelinkova, B. Rihova, Polymeric Drugs Based on Conjugates of Synthetic and Natural Macromolecules I. Synthesis and Physico-chemical Characterisation. J. Controlled Rel. 64, 63-79 (2000*)*
K. Ulbrich, T. Etrych, P. Chytil, M. Jelinkova, B. Rihova, HPMA Copolymers with pH-Controlled Release of Doxorubicin, In vitro Cytotoxicity and in vivo Antitumor Activity. J. Controlled Release 87, 33-47 (2003)
K. Ulbrich, T. Etrych, P. Chytil, M. Jelinkova, B. Rihova, Antibody-Targeted Polymer-Doxorubicin Conjugates with pH-Controlled Activation, J. Drug Targeting 12(8) (2004) 477-489*].* (A)
K. Ulbrich, V. Subr, Polymeric Anticancer Drugs with pH-Controlled Activation, Adv. Drug Delivery Rev. 56/7, 1025-1052 (2004*)* (B)
K. Ulbrich, T. Etrych, B. Rihova, M. Jelinkova, M. Kovar: pH Sensitive polymeric conjugates of an anthracycline cancerostatic for targeted therapy. CZ 293 787*,* CZ 293886

## Claims

1. A polymeric drug in the form of a conjugate of a copolymer of N-(2-hydroxypropyl)methacrylamide HPMA with doxorubicin, bound to the polymer by means of spacers containing hydrolytically cleavable hydrazone bonds, of formula I 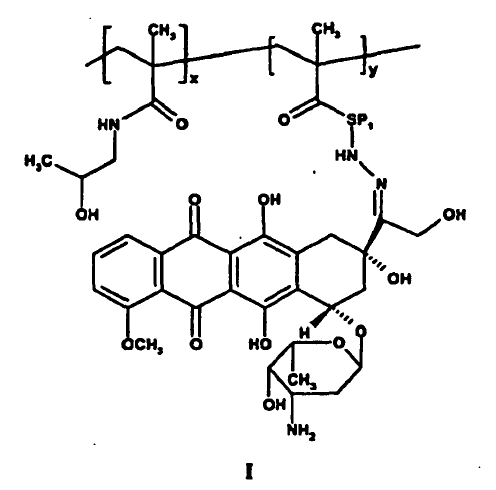 wherein SP₁ represents an aminoacyl spacer selected among the glycyl, glycylglycyl, βalanyl, 6-aminohexanoyl, 4-aminobenzoyl groups or a complex acyl derived from the oligopeptides GlyPheGly, GlyLeuGly, GlyLeuPheGly or GlyPheLeuGly, x = 40 to 335, y
= 1 to 25.
consisting of from 90 to 99.5 mol. % of HPMA units and from 10 to 0.5 mol. % of doxorubicin-containing comonomeric units.

2. A method for the preparation of the polymeric conjugate of formula I according to claim *1, **characterized by*** direct copolymerization of the doxorubicin-containing monomer of formula II 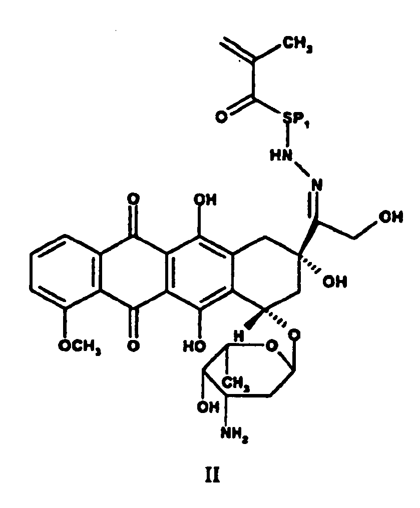 wherein SP₁ is as defined in claim 1,
with HPMA in a molar ratio of 90 to 99.5 : 10 to 0.5.

3. The method according to claim 2, ***characterized in* that** polymerization is performed in the medium of methanol, ethanol, dimethylsulfoxide or dimethylformamide and is initiated with heat-degradable radical polymerization initiators.

4. The method according to claim 3, ***characterized in* that** the radical polymerization initiators are selected from the group consisting of azobis(isobutyronitrile) AIBN, azobis(isocyanovaleric acid ABIC and diisopropylpercarbonate DIP.

5. The method according to claim 4, ***characterized in* that** the initiator is selected from the group of AIBN or ABIC and the reaction takes place at 50 to 60 °C for 15 to 24 hours.

6. The method according to claim 4, ***characterized in* that** DIP is selected as the initiator and the reaction takes place at 40 to 50 °C for 15 to 24 hours.

7. The method according to claims 2 to 6, ***characterized in* that** the monomeric unit of formula II is prepared by reaction of doxorubicin hydrochloride with methacryloyl(aminoacyl)hydrazines of formula MA-SP₁NHNH₂, wherein MA is methacryloyl and SP₁ is as defined in claim 1, in an organic solvent in the presence of acetic acid.

8. The method according to claim 7, ***characterized in* that** in the starting mixture, the concentration of doxorubicin is selected in the range of from 10 to 30 mg/ml and the concentration of acetic acid ranges is selected in the range of from 35 to 60 mg/ml.

9. The method according to claim 8, ***characterized in* that** in the starting reaction mixture, the concentration of doxorubicin hydrochloride is 19 mg/ml and that of acetic acid is 51 mg/ml.

10. The method according to any of claims 7 to 9, ***characterized in* that** the reaction is performed at a temperature of 20 to 35 °C for 20 to 28 hours.

11. The method according to claim 10, ***characterized in* that** the reaction is performed at 25 °C for 24 hours.

12. The method according to claims 7 to 11, ***characterized in* that,** after completion of the reaction, a copolymer of HPMA with methacryloylated(aminoacyl)hydrazide is used to remove excess doxorubicin.

13. The method according to claims 7 to 12, ***characterized in* that** the reaction medium is constituted by an organic solvent selected from the group consisting of methanol, anhydrous ethanol, dimethylsulfoxide, dimethylformamide and dimethylacetamide.

14. The method according to claim 13, ***characterized in* that** the reaction medium is constituted by methanol.

## Patentansprüche

1. Polymeres Arzneimittel in Form eines Copolymer-Konjugats von N-(2-Hydroxypropyl)methacrylamid (HPMA) mit Doxorubicin, das durch Spacer, enthaltend hydrolytisch spaltbare Hydrazonbindungen, an das Polymer gebunden ist, mit der Formel (I): 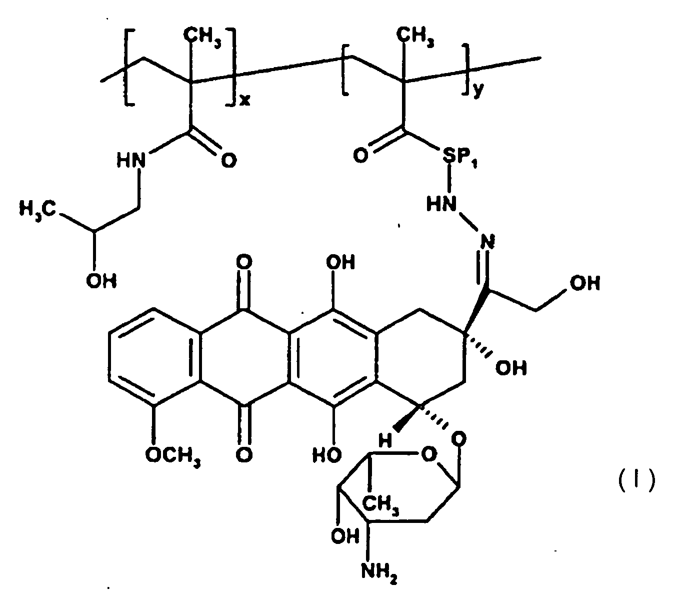 worin SP₁ einen Aminoacryl-Spacer, ausgewählt aus Glycyl, Glycylglycyl, β-Alanyl, 6-Aminohexanoyl, 4-Aminobenzoylgruppen oder einem komplexen Acyl, das von den Oligopeptiden GlyPheGly, GlyLeuGly, GlyLeuPheGly oder GlyPheLeuGly abgeleitet ist, darstellt, x = 40 bis 335 ist und y = 1 bis 25 ist, bestehend aus 90 bis 99,5 mol-% HPMA-Einheiten und 10 bis 0,5 mol-% Doxorubicin enthaltenden Comonomer-Einheiten.

2. Verfahren zur Herstellung eines polymeren Konjugats der Formel (I) gemäss Anspruch 1, **gekennzeichnet durch** direkte Copolymerisation des Doxorubicin enthaltenden Monomers der Formel (II): 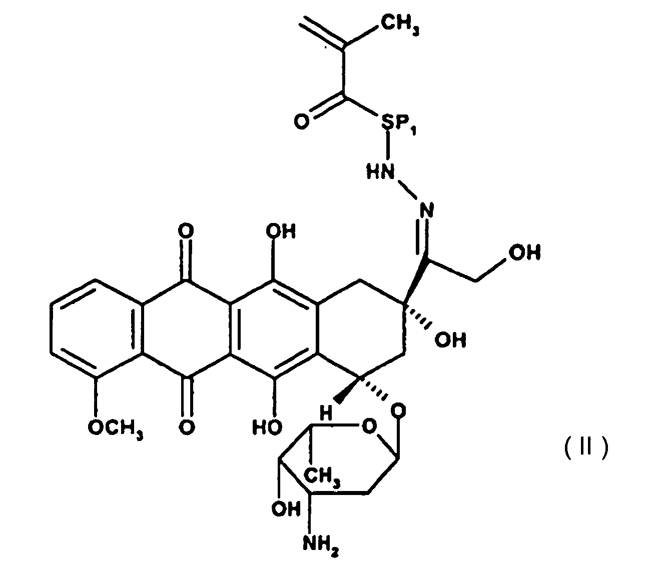 worin SP₁ wie in Anspruch 1 definiert ist, mit HPMA in einem molaren Verhältnis von 90 bis 99,5 : 10 bis 0,5.

3. Verfahren gemäss Anspruch 2, **dadurch gekennzeichnet, dass** die Polymerisation in dem Medium Methanol, Ethanol, Dimethylsulfoxid oder Dimethylformamid durchgeführt und mit wärmeabbaubaren Radikalpolymerisationsinitiatoren initiiert wird.

4. Verfahren gemäss Anspruch 3, **dadurch gekennzeichnet, dass** die Radikalpolymerisationsinitiatoren aus der Gruppe bestehend aus Azobis(isobutyronitril) (AIBN), Azobis(isocyanovaleriansäure) (ABIC) und Diisopropylpercarbonat (DIP) ausgewählt sind.

5. Verfahren gemäss Anspruch 4, **dadurch gekennzeichnet, dass** die Initiatoren aus der Gruppe AIBN oder ABIC ausgewählt sind und die Reaktion für 15 bis 24 Stunden bei 50 bis 60°C stattfindet.

6. Verfahren gemäss Anspruch 4, **dadurch gekennzeichnet, dass** DIP als Initiator ausgewählt wird und die Reaktion für 15 bis 24 Stunden bei 40 bis 50°C stattfindet.

7. Verfahren gemäss Ansprüchen 3 bis 6, **dadurch gekennzeichnet, dass** die Monomer-Einheit der Formel (II) durch Umsetzen von Doxorubicin-Hydrochlorid mit Methacryloyl(aminoacyl)hydrazinen der Formel MA-SP₁NHNH₂, worin MA Methacryloyl ist und SP₁ wie in Anspruch 1 definiert ist, in einem organischen Lösungsmittel in Gegenwart von Essigsäure hergestellt wird.

8. Verfahren gemäss Anspruch 7, **dadurch gekennzeichnet, dass** in der Ausgangsmischung die Konzentration an Doxorubicin im Bereich von 10 bis 30 mg/ml und die Konzentration an Essigsäure im Bereich von 35 bis 60 mg/ml ausgewählt wird.

9. Verfahren gemäss Anspruch 8, **dadurch gekennzeichnet, dass** in der Ausgangsreaktionsmischung die Konzentration an Doxorubicin-Hydrochlorid 19 mg/ml und die an Essigsäure 51 mg/ml beträgt.

10. Verfahren gemäss irgendeinem der Ansprüche 7 bis 9, **dadurch gekennzeichnet, dass** die die Reaktion für 20 bis 28 Stunden bei einer Temperatur von 20 bis 35°C durchgeführt wird.

11. Verfahren gemäss Anspruch 10, **dadurch gekennzeichnet, dass** die Reaktion für 24 Stunden bei 25°C durchgeführt wird.

12. Verfahren gemäss Ansprüchen 7 bis 11, **dadurch gekennzeichnet, dass** nach Beendigung der Reaktion ein Copolymer von HPMA mit methacryloyliertem (Aminoacyl)hydrazid zur Entfernung des überschüssigen Doxorubicins verwendet wird.

13. Verfahren gemäss Ansprüchen 7 bis 12, **dadurch gekennzeichnet, dass** das Reaktionsmedium durch ein organisches Lösungsmittel, ausgewählt aus der Gruppe bestehend aus Methanol, wasserfreiem Ethanol, Dimethylsulfoxid, Dimethylformamid und Dimethylacetamid, gebildet wird.

14. Verfahren gemäss Anspruch 13, **dadurch gekennzeichnet, dass** das Reaktionsmedium durch Methanol gebildet wird.

## Revendications

1. Un médicament polymère sous forme d'un conjugué de copolymère de *N-(2*-hydroxypropyl)-méthacrylamide HPMA et de doxorubicine, liée au polymère par des moyens d'espacement contenant des liaisons hydrazone clivables par voie hydrolytique, de formule I 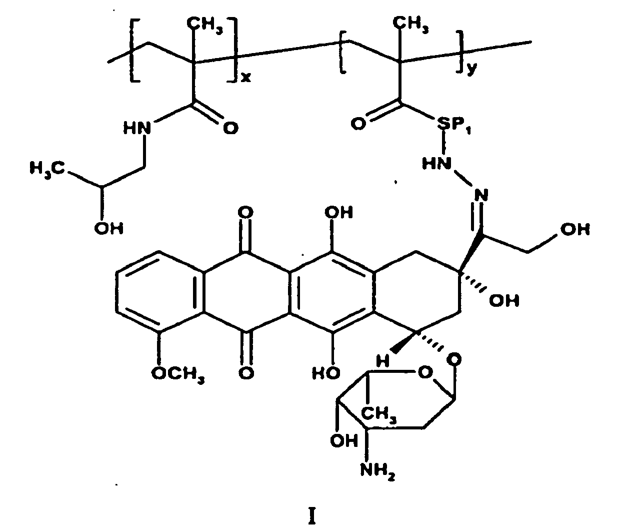 dans laquelle
- SP₁ représente un moyen d'espacement consistant en un aminoacyl choisi dans le groupe comprenant les radicaux glycyl, glycylglycyl, β-alanyl, 6-aminohéxanoyl, 4-aminobenzoyl ou un complexe acyl dérivant des oligopeptides GlyPheGly, GlyLeuGly, GlyLeuPheGly ou GlyPheLeuGly,
- x = 40 à 335,
- y = 1 à 25,
consistant en 90 à 99,5 mol.% d'unités de HPMA et de 10 à 0,5 mol.% d'unités de comonomères contenant de la doxorubicine.

2. Un procédé pour la préparation du conjugué polymère de formule I, selon la revendication 1, **caractérisé par** la copolymérisation directe du monomère contenant la doxorubicine de formule II, dans laquelle SP₁ est tel que défini dans la revendication 1, 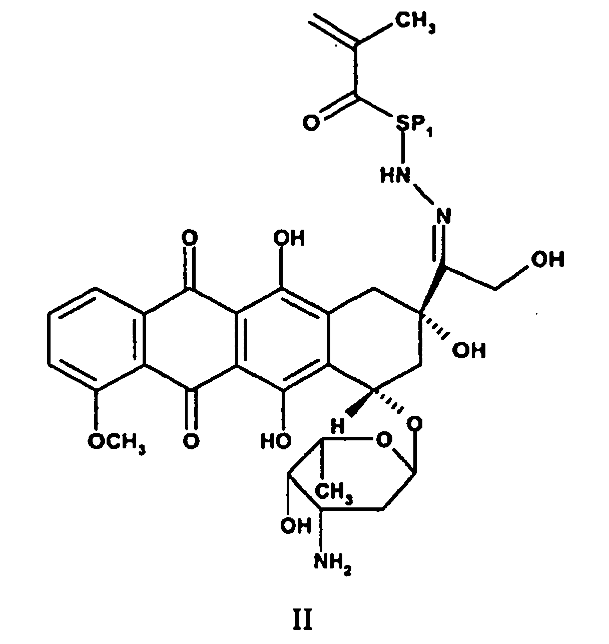 avec HPMA en un rapport molaire de 90 à 99,5/10 à 0,5.

3. Le procédé selon la revendication 2, **caractérisé en ce que** la polymérisation est mise en oeuvre dans un milieu consistant en méthanol, éthanol, diméthylsulfoxyde ou diméthylformamide et est initiée par action d'initiateurs de polymérisation radicalaire thermo-dégradables.

4. Le procédé selon la revendication 3, **caractérisé en ce que** les initiateurs de polymérisation radicalaire sont choisis dans le groupe consistant en l'azobis-(isobutyronitrile) AIBN, l'acide azobis-(isocyanovalérique) ABIC et le diisopropylpercarbonate DIP.

5. Le procédé selon la revendication 4, **caractérisé en ce que** l'initiateur est choisi dans le groupe AIBN ou ABIC et **en ce que** la réaction est réalisée entre 50 et 60°C pendant 15 à 24 heures.

6. Le procédé selon la revendication 4, **caractérisé en ce que** DIP est choisi en tant qu'initiateur et **en ce que** la réaction est réalisée entre 40 et 50°C pendant 15 à 24 heures

7. Le procédé selon les revendications 2 à 6, **caractérisé en ce que** l'unité monomère de formule Il est préparé par réaction de chlorhydrate de doxorubicine avec des méthacryloyl-(aminoacyl)-hydrazines de formule MA-SP₁NHNH₂, dans laquelle MA est le radical méthacryloyl et SP₁ est tel que défini dans la revendication 1, dans un solvant organique en présence d'acide acétique.

8. Le procédé selon la revendication 7, **caractérisé en ce que** dans le mélange de départ, la concentration de doxorubicine est choisie dans la gamme alant de 10 à 30 mg/ml et la concentration en acide acétique est choisie dans la gamme allant de 35 à 60 mg/ml.

9. Le procédé selon la revendication 8, **caractérisé en ce que** dans le mélange réactionnel de départ, la concentration en chlorhydrate de doxorubicine est de 19 mg/ml et la concentration en acide acétique est de 51 mg/ml.

10. Le procédé selon une quelconque des revendications 7 à 9, **caractérisé en ce que** la réaction est réalisée à une température of 20 à 35°C pendant 20 à 28 heures.

11. Le procédé selon la revendication 10, **caractérisé en ce que** la réaction est réalisée à 25°C pendant 24 heures.

12. Le procédé selon les revendications 7 à 11, **caractérisé en ce que**, après achèvement de la réaction, un copolymère de HPMA avec un méthacryloyl (aminoacyl)-hydrazide est employé pour éliminer l'excès de doxorubicine.

13. Le procédé selon les revendications 7 à 12, **caractérisé en ce que** le milieu dans lequel la réaction est effectuée consiste en un solvant organique choisi dans le groupe consistant en méthanol, éthanol anhydre, diméthylsulfoxyde, diméthylformamide et diméthylacétamide.

14. Le procédé selon la revendication 13, **caractérisé en ce que** le milieu dans lequel la réaction est effectuée consiste en méthanol.
